# EUROPEAN PATENT SPECIFICATION

(11) **EP 3 920 996 B1**
(45) Date of publication and mention of the grant of the patent: **11.09.2024**
(21) Application number: 20720247.4
(22) Date of filing: 25.03.2020
(51) Int. Cl.: A61M 1/14, A61M 1/34, G16H 20/40, G16H 40/63

(54) **KEYBOARD FOR A BLOOD TREATMENT MACHINE**
TASTATUR FÜR BLUTBEHANDLUNGSMASCHINE
CLAVIER POUR MACHINE DE TRAITEMENT DU SANG

(30) Priority: 28.03.2019 US 201916367411
(43) Date of publication of application: 15.12.2021
(73) Proprietor: Fresenius Medical Care Holdings, Inc., Waltham, MA 02451-1457 (US); Fresenius Medical Care Deutschland GmbH, 61352 Bad Homburg (DE)
(72) Inventor: CRNKOVICH, Martin Joseph, Waltham, MA 02451-1457 (US); WEAVER, Colin, Waltham, MA 02451-1457 (US); SCHLAEPER, Christian, 61352 Bad Homburg (DE); LAU, Su Ting, Waltham, Massachusetts 02451-1457 (US)
(74) Representative: Fish & Richardson P.C.
(86) International application number: PCT/US2020/024697
(87) International publication number: WO 2020/198348

(56) References cited:
- US-A1- 2009 309 835
- US-A1- 2013 053 651
- US-A1- 2016 370 911
- US-A1- 2017 045 947

## Description

### TECHNICAL FIELD

This disclosure relates to a keyboard for a blood treatment machine.

### BACKGROUND

During hemodialysis ("HD"), the patient's blood is passed through a dialyzer of a dialysis machine while also passing a dialysis solution or dialysate through the dialyzer. A semi-permeable membrane in the dialyzer separates the blood from the dialysate within the dialyzer and allows diffusion and osmosis exchanges to take place between the dialysate and the blood stream. These exchanges across the membrane result in the removal of waste products, including solutes like urea and creatinine, from the blood. These exchanges also regulate the levels of other substances, such as sodium and water, in the blood. In this way, the dialysis machine acts as an artificial kidney for cleansing the blood.

During peritoneal dialysis ("PD"), a patient's peritoneal cavity is periodically infused with dialysis solution or dialysate. The membranous lining of the patient's peritoneum acts as a natural semi-permeable membrane that allows diffusion and osmosis exchanges to take place between the solution and the blood stream. These exchanges across the patient's peritoneum, like the continuous exchange across the dialyzer in HD, result in the removal waste products, including solutes like urea and creatinine, from the blood, and regulate the levels of other substances, such as sodium and water, in the blood.

Many PD machines are designed to automatically infuse, dwell, and drain dialysate to and from the patient's peritoneal cavity. The treatment typically lasts for several hours, often beginning with an initial drain cycle to empty the peritoneal cavity of used or spent dialysate. The sequence then proceeds through the succession of fill, dwell, and drain phases that follow one after the other. Each phase is called a cycle. Data can be input into HD and PD machines in various ways. In some cases, a keyboard or laptop computer is connected to the machine and used to input data into the machine.

US 2016/0370911 describes a medical fluid treatment machine comprising: a pump configured to pump medical fluid to and from a patient; an input device configured to: display touch buttons, at least some of the touch buttons each representing a grapheme, and detect haptic interactions between a user and the touch buttons, wherein an interaction causes information related to the touch button to be received by the medical fluid treatment machine; and a control unit configured to control the input device, wherein controlling the input device includes causing the input device to switch between displaying i) a first set of touch buttons that correspond to a first language, and ii) a second set of touch buttons that correspond to a second language.

US 2009/0309835 describes processing devices in the medical device area.

US 2017/0045947 describes a dialysis machine that includes a processing unit configured to transmit control data; a pump configured to pump medical fluid to and from a patient based at least in part on control data received from the processing unit; an electronic panel comprising: a display surface, and at least one panel control unit configured to cause the electronic panel to display at least one user interface element that can be invoked by a user; at least one projector; and at least one camera; wherein the one or more processing units are configured to: process input received by the camera, determine a location of a physical object in a field of view of the camera based on the processed input, determine, based on processed input received on at least one occasion, that the location of the physical object represents an invocation of the at least one user interface element displayed on the electronic panel, and determine the control data based on the processed input.

US 2013/0053651 describes a method performed on a dialysis machine that includes receiving a first value that represents a pre-dialysis body weight of a patient and receiving a second value that represents a volume of liquid inside a body of the patient. The method also includes administering dialysis to the patient, calculating an instantaneous body weight of the patient based at least in part on the first value, determining a length of time for which the dialysis was administered to the patient, determining a dialyzer clearance associated with the dialysis, and calculating, based at least in part on the length of time, the instantaneous body weight, the volume, and the dialyzer clearance, a urea reduction ratio associated with the dialysis.

### SUMMARY

In some aspects, a blood treatment machine includes a user interface configured to display information, a transceiver configured to send and receive signals, a controller configured to process input signals received by the transceiver and display information on the user interface, one or more pumps, and a first door. The first door has a closed configuration in which the door covers the one or more pumps and an open configuration in which the one or more pumps are accessible by a user. The blood treatment machine further includes at least a portion of a keyboard mounted on the door, the keyboard being configured to transmit signals to the transceiver of the machine.

In some embodiments, the blood treatment machine comprises a surface on which the one or more pumps are disposed.

In some embodiments, the door covers the surface.

In some embodiments, the door is a first door and the blood treatment machine further comprises a second door.

In some embodiments, the portion of the keyboard mounted on the door is a first portion mounted on the first door and the keyboard has a second portion mounted on the second door.

In some embodiments, the first portion of the keyboard and the second portion of the keyboard are wirelessly connected.

In some embodiments, the first portion of the keyboard comprises a signal transceiver, a battery, and a controller and the second portion of the keyboard comprises a signal transceiver and a battery.

In some embodiments, the transceiver of the first portion is configured to send signals to the transceiver of the blood treatment machine.

In some embodiments, the keyboard is bolted onto the door.

In some embodiments, the keyboard is mounted on a bracket.

In some embodiments, the door comprises a recess and a ledge that defines a portion of the recess, wherein the keyboard is mounted on the ledge.

In some embodiments, the keyboard is wirelessly connected to the machine.

In some embodiments, the keyboard is electronically connected to the machine using wired connections.

In some embodiments, signal receiver is releasably connected to the machine.

In some embodiments, the signal receiver connected to the machine by a USB port.

In some embodiments, the keyboard is configured to transmit signals to the transceiver of the machine using bluetooth.

In some embodiments, the keyboard is on a front face of the door, accessible to a user in the closed configuration.

In some aspects, a system in which a blood treatment machine is configured to communicate with a remote server. The system includes the blood treatment machine. The blood treatment machine includes a first door for covering components of the blood treatment machine, a controller for controlling the blood treatment machine, a signal receiver for receiving signals from electronically connected devices, and a user interface controlled by the controller for displaying information. The door has an open position and a closed position The blood treatment machine further includes a keyboard at least partially mounted on the first door of the blood treatment machine and electronically connected to the blood treatment machine. The keyboard is configured to send user input signals to the signal receiver of the blood treatment machine. The system further includes the remote server in connection with the blood treatment machine. The remote server contains patient information. The blood treatment machine is configured to retrieve patient information from the remote server using inputs received from the keyboard.

In some embodiments, the remote server and machine are in wireless connection and the keyboard and the machine are in wireless connection.

Embodiments can include one or more of the following advantages. Mounting or integrating a keyboard with one or more doors of the blood treatment machine can improve an operator's access to the blood pumps on the face of the machine. Operators frequently connect external keyboards to the dialysis machine to input patient data and treatment notes. To access the bloodlines, the operators must move the keyboards out of the way. In some cases, those keyboards are placed in front of the dialysis machine. In such cases, these external keyboards may be missed during routine cleaning. Mounting the keyboard on the door of the machine helps to ensure that that the keyboard is easily removed from the workspace when accessing the bloodlines and pumps on the face of the machine. Because the keyboard is mounted to or integrated in the door(s) of the machine, for example, the mere act of opening the door to access the bloodlines and pumps will move the keyboard out of the way. This is particularly advantageous during emergency events when quick action is necessary. Further, the keyboard may be cleaned more regularly, as part of the machine cleaning and will not be moved onto potentially unclean surfaces adjacent to the blood treatment machine, thus reducing cross contamination risks. The keyboard may also increase the ease of data entry as the keyboard is directly in front of the machine, thus allowing the operator to easily take notes during, before, or after blood treatment.

The details of one or more embodiments are set forth in the accompanying drawings and the description below. Other features, and advantages will be apparent from the description and drawings, and from the claims. The invention is defined by the appended claims.

### BRIEF DESCRIPTION OF THE FIGURES

Fig. 1 illustrates a blood treatment system with a split keyboard attached to doors in a closed position.
Fig. 2 illustrates the blood treatment system of Fig. 1 with the split keyboard attached to doors in an open position.
Fig. 3 is a side view of the blood treatment system of Fig. 1 with the doors in the closed position.
Fig. 4 is an enlarged view of the split keyboard of the blood treatment system of Fig. 1.
Fig. 5 is schematic of a system including the blood treatment machine of Fig. 1 in wireless communication with a split keyboard and a server.
FIG 6 is a split keyboard with a jagged edge.
Fig. 7A is a top view of a split keyboard designed to provide haptic feedback.
Fig. 7B is a side view of the split keyboard of FIG 7A.
Fig. 8 is a split keyboard with a touchpad.
Fig. 9A is a front view of a blood treatment system with a split keyboard attached to the doors.
Fig. 9B is a side view of the blood treatment system of Fig. 9A with the bolted split keyboard attached to the doors.
Fig. 10A-C is a front view of a blood treatment system with a hinged split keyboard.
Figs. 10B and 10C illustrate the blood hinged split keyboard of the blood treatment system in Fig. 10A in the expanded and the folded position, respectively.
Fig. 11 is a blood treatment system with an adjustable height split keyboard.
Fig. 12 illustrates a front view of a blood treatment system of with a keyboard on a single door in a closed position.
Fig. 13 is a front view of the blood treatment system of Fig. 12 with the keyboard on the door in an open position.
Fig. 14 is a side view of the blood treatment system of Fig. 12 with the door in the closed position.
Fig. 15 is an enlarged view of the keyboard of the blood treatment system of Fig. 12.

Like reference symbols in the various drawings indicate like elements.

### DETAILED DESCRIPTION OF THE FIGURES

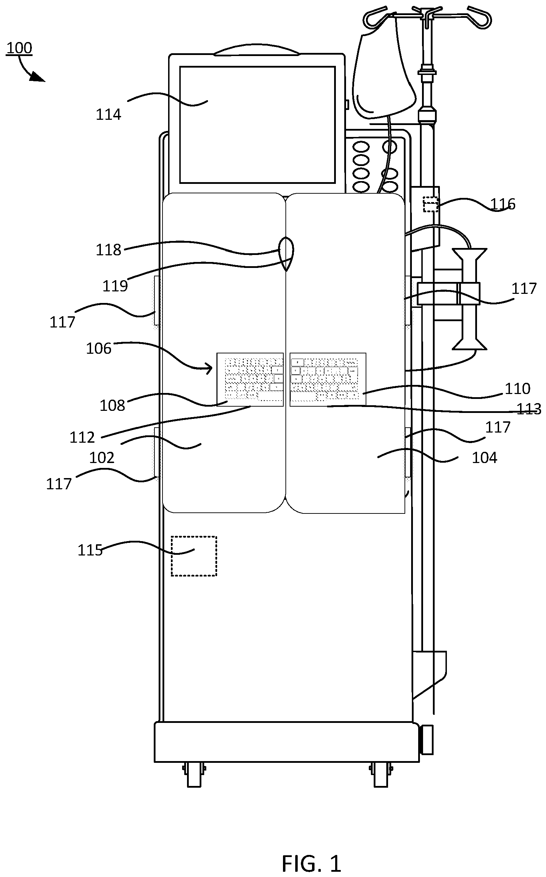

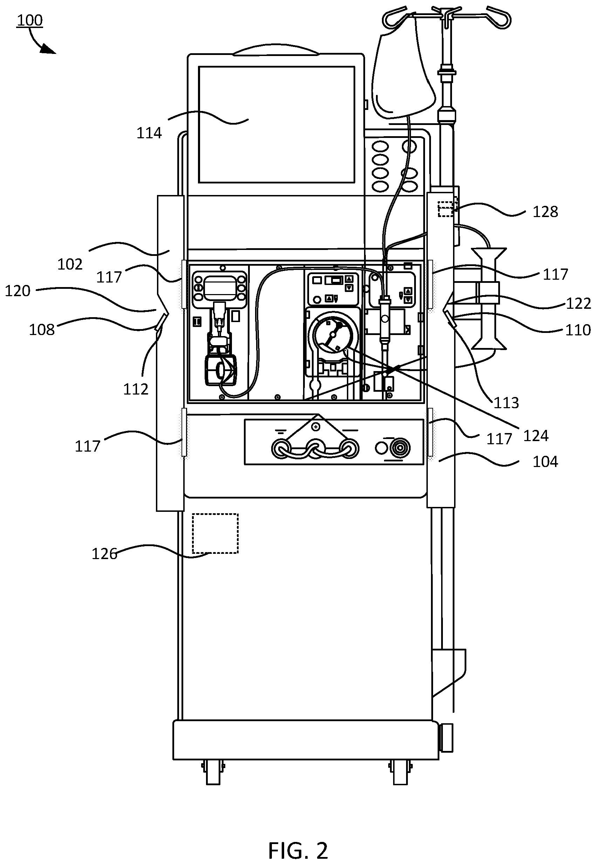

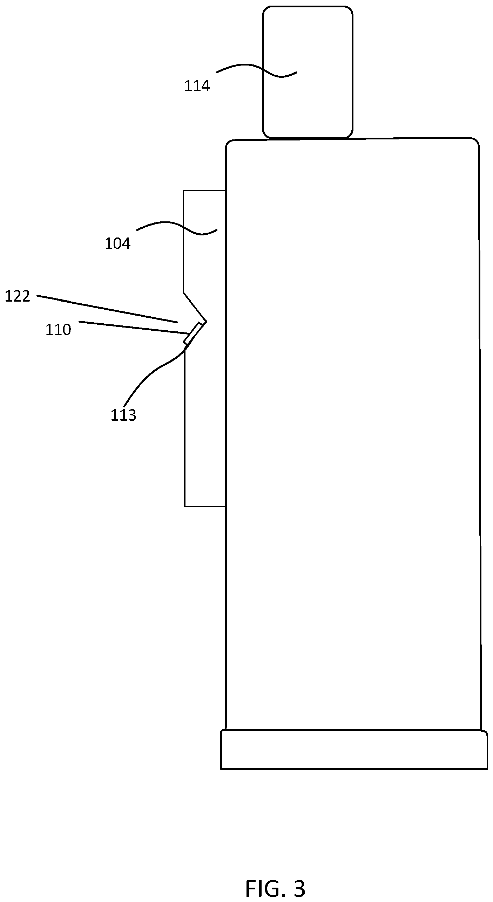

Fig. 1 shows a blood treatment machine 100 with a first door 102 and a second door 104. The first and second doors 102, 104 are in a closed configuration in which the doors 102, 104 cover pumps (e.g. blood pumps, dialysate pumps, ultrafilration umps, etc.) that are used in blood treatment. Fig. 1 also shows a split keyboard 106 with a first portion 108 and a second portion 110. The first portion 108 of the keyboard 106 is mounted on the first door 102. The second portion 110 of the keyboard 106 is mounted on the second door 104. The keyboard 106 is in wireless communication with the blood treatment machine 100. As shown in Figs. 2 and 3, the doors 102, 104 have ledges 112, 113 that extend towards the machine 100 to define the lower end of a recess. The first portion 108 of the keyboard 106 is mounted on the ledge 112 of the first door 102 and the second portion 110 is mounted on the ledge 113 of the second door 104. During, before, or after blood treatment, an operator may use the split keyboard 106 to input information related to the treatment. The keyboard 106 may be used, for example, to input notes on the blood treatment, prescription data, patient ID, etc. The machine also includes a user interface 114 that displays information about the treatment, a controller 115 that controls the blood treatment, and a signal transceiver 116 that sends and receives signals.

Still referring to Fig. 1, the first door 102 is connected to the machine 100 by hinges 117, such that the door swings along a vertical axis about the hinge 117. The second door 104 is connected to the machine 100 in the same manner as the first door 102, using hinges 117. Handle 118 on the first door 102 and handle 119 on the second door 104 aid in rotating the doors about their respective axes. The handle may be a simple knob, a rotating handle, or a sliding handle. The doors 102, 104 can be made of a transparent material, such as a transparent plastic. Transparent doors allow an operator to view the blood pumps and blood lines when the doors are closed. The door handles 118, 119 may include latches that secure the doors in the closed position and latch sensors (not shown) that connect to a controller on the machine 100. The latch sensor with the controller can determine whether the doors 102, 104 are in an open position or the closed position.

Fig. 2 shows the machine 100 with the doors 102, 104 in the open position. In the open position, the pumps and other components on the face of the machine 100 are exposed and accessible to an operator. In the open position, the machine 100 will not perform blood treatment. The latch sensor, signals whether latches on the doors 102, 104 are engaged or disengaged and in turn indicates whether the doors 102, 104 are open or closed. In the open position the latch is disengaged and in the closed position the latch is engaged. There can be two latch sensors (not shown), one latch sensor for the first door 102 and one latch sensor for the second door 104.

The doors move from the closed position to the open positon, as shown in Fig. 2, by rotating about the hinges 117. A first recess 120 is partially defined by the ledge 112 of the first door 102. The second door 104 also has a second recess 122 partially defined by the ledge 113. The recess is sized to at least partially receive the hands of the operator. The first portion 108 of the keyboard 106 is mounted on the ledge 112 of the first portion 102. The second portion 110 of the keyboard 106 is mounted on the ledge 113 of the second door 104.

The doors 102, 104, as shown in Figs. 1 and 2, are able to repeatedly move from a closed position to an open position and vice versa. As the first door 102 moves, the first portion 108 of the split keyboard 106 moves with it. Similarly, as the second door 104 moves, the second portion 110 of the split keyboard 106 moves with it. Movement of the doors 102, 104 prevents or allows access to blood pumps 124 of the machine 100.

Fig. 3 shows a side view of the machine 100. The first and second doors 102,104 are in the closed position and the second door 104 is also in the closed position. As both doors 102, 104 are closed, the machine 100 may begin or commence blood treatment. The second recess 122 at least partially defined by the ledge 113 can be easily observed in the side view. The recesses 120, 122 and the ledges 113 align so that the first portion 108 and the second portion 110 are mounted at the same angle and height. In this way, the first and second portions 108, 110 cooperate to function as a single keyboard when the doors 102, 104 are closed.

The recesses 120, 122 are sized to receive hands of the operator for typing. Typing applies repeated forces onto a keyboard, in addition to the weight of the keyboard and the weight of the hands of the operator. The keyboard 106 is supported by mounting the keyboard 102 on the ledge in the recesses 120, 122 of the doors 102, 104. In such a configuration, the keyboard 106 does not sag over time due to the repeatedly applied forces.

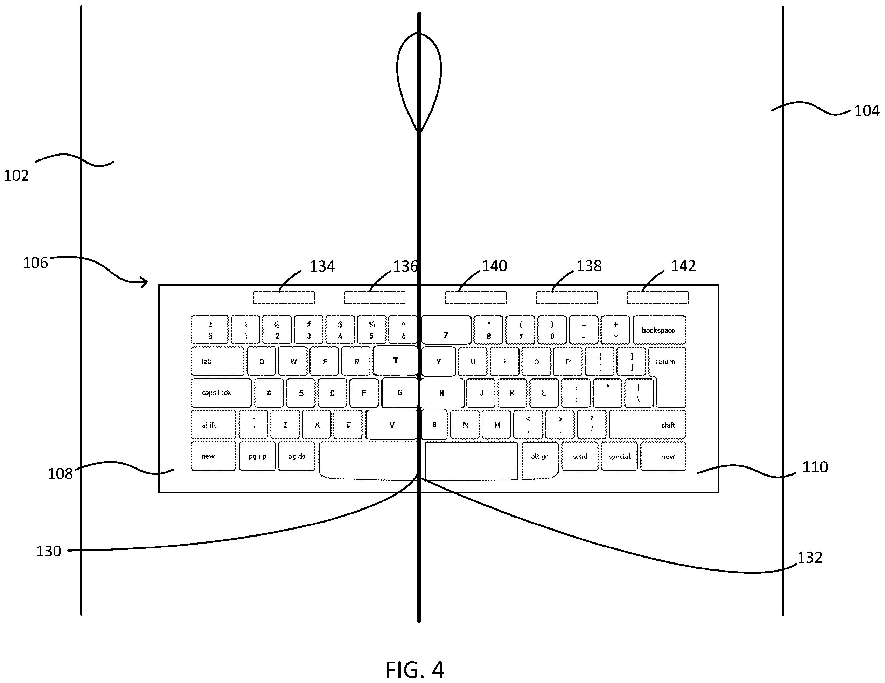

Fig. 4 shows an enlarged view of the keyboard 106 mounted on the first door 102 and the second door 104. The keyboard 106 is split evenly down the middle of a standard keyboard layout. The keyboard 106 is evenly split so that the first portion 108 and the second portion 110 generally have the same number of keys. Other keyboards may be split so that either the first portion has more keys than the second portion or the second portion has more keys than the first portion. To prevent a toothed edge, the T, G, and V keys on the first portion 108 of the keyboard 106 are slightly extended towards an edge 130 of the first portion 108. Similarly, keys 7, Y, and H are extended towards an edge 132 of the second portion 110.

Fig. 4 also shows electronics housed within the split keyboard 106. The first portion 108 of the keyboard 106 includes a battery 134 and a signal transceiver 136. The second portion 110 of the keyboard 106 includes a battery 138, a signal transceiver 140, and a processor 142. The transceiver 136 of the first portion 108 is in wireless electronic communication with the transceiver 140 of the second portion 110. The transceiver 140 of the second portion 110 is also in wireless communication with the transceiver 116 of the machine 100.

In use, an operator types information into the machine 100 using the keyboard 106. The user may type using keys from the first portion 108, second portion 110, or most commonly, a combination of the keys on the first portion 108 and on the second portion 110. When the user presses a key from the first portion 108, a keystroke signal is sent from the transceiver 136 to the transceiver 140 on the second portion 110 of the keyboard 106. The keystroke signal is then sent from the transceiver 140 on the second portion to the transceiver 116 on the machine. The controller 115 of the machine processes the keystroke signal and displays the character associated with the keystroke signal on the user interface 114.

The processor 142 of the second portion 110 is used to ensure that the keystroke signals being sent to the machine 100 are sent in the correct order. For example, if the user presses "a" on the first portion 108 and "1" on the second portion 110, after the "a" keystroke, the processor ensures that the "a" keystroke signal is sent prior to the "1" keystroke signal. One way to ensure proper order of keystrokes is to add a timestamp to the keystroke so that the processor 142 may put the keystroke signals in chronological order. The processor 142 may also be used to determine keystroke combinations, for example "shift" + "a" indicates a capitalized "A" or "ctrl"+ "c" to copy a string of characters.

Prior to a treatment, the user connects a blood line set to the pumps and other components on the face of the machine and then connects patient lines of the blood line set to the patient. The doors are in an open position to access the pumps 124 and other components on the face of the machine, as shown in Fig. 2. In this configuration the blood treatment machine is prevented from proceeding with blood treatment. After connecting the blood line set, the operator shuts the doors 102, 104 so that the doors 102, 104 latch. In this configuration the blood treatment may proceed.

The operator may use the keyboard to input prescription data or patient data, such as blood pressure, patient weight, and preliminary notes. Blood treatment commences and the patient's blood is cleaned over a period of time.

During blood treatment it may be useful to add notes to the patient file. For example, the operator can input notes regarding patient complications, unusual events, etc. To do this, a user may select the notes section of the user interface 114 and type using the split keyboard 106, easily accessible on the doors 102, 104 of the machine 100.

If an event occurs in which the operator needs to access the face of the machine 100, for example, reconnecting or checking the blood lines, the operator opens the doors 102, 104. Opening the doors 102, 104 moves the split keyboard 106 away from the pumps of the machine 100, making a clear and uncluttered workspace for the operator to address the event. Because the keyboard portions 108, 110 are attached to the doors 102, 104, the do not need to be moved out of the way prior to opening the doors 102, 104.

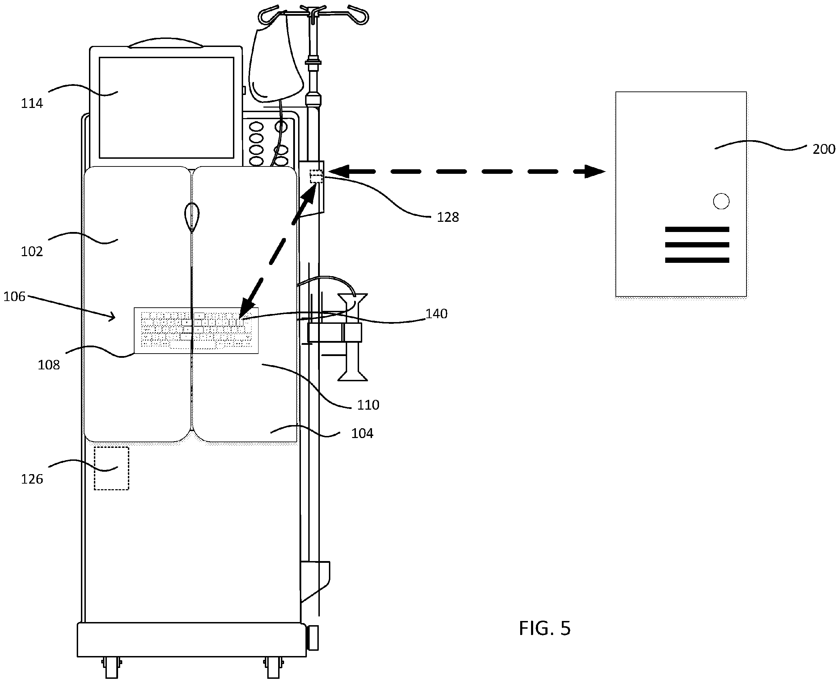

Figure 5 shows the machine 100 in communication with a remote server 200 and in communication with the keyboard 106. The remote server 200 contains patient data that may be used during blood treatment. Patient data may be, for example, previous treatment data, prescription data, and/or medical history data. The machine 100 is in wireless connection with the server 200 and can write notes, change data, or add data to the exiting patient file. To do this, an operator inputs characters using the keyboard 106. The transceiver 140 of the keyboard, sends the character signals to the transceiver 116 on the machine 100. The controller 115 of the machine then processes the signals and generates text on the user interface 114. This text on the user interface may be saved to the patient file on the server by sending the amended file from the transceiver of the machine 100 to the remote server 200.

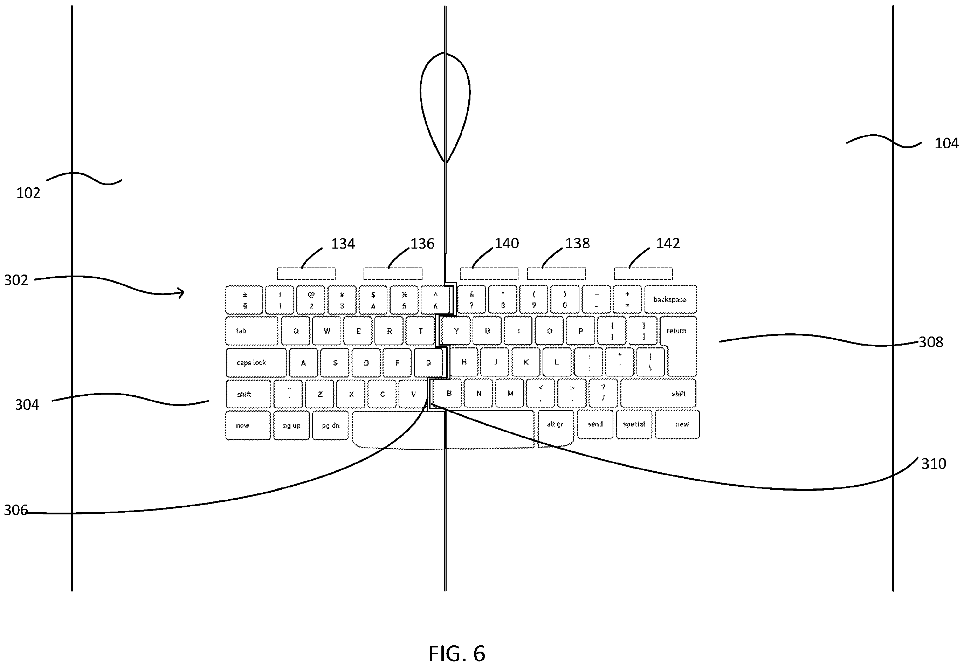

While the split keyboard included in the system of Figs. 1-4 includes a smooth connecting edge, other types of keyboards can be used. Fig. 6, for example, shows a jagged split keyboard 302 that has a standard keyboard layout. The keyboard 302 has a first portion 304 with an inner edge 306 and a second portion 308 with an inner edge 310. The keys of the first portion 304 end at the inner edge 306 in a toothed pattern. Similarly, the keys of the second portion 308 at the edge 310 are in a jagged or toothed pattern. The keyboard 302 in Fig. 6 differs from keyboard 106 because both portions 108, 110 of the keyboard 106 in Figs. 1-4 have extended keys to prevent the toothed formation. The keyboard 302 is split so that the size of the first portion 304 is generally equivalent to the size of the second portion 308. However, the keyboard may be split at other locations in alternative embodiments. The keyboard 302 includes the same electronics as the keyboard 106 and is configured to operate in the same manner.

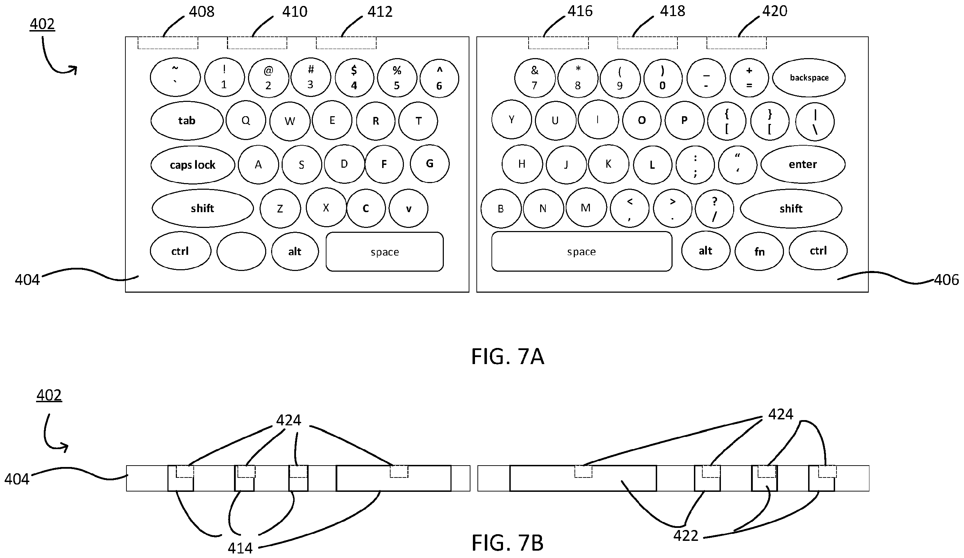

Other types of keyboards can also be used with the systems described herein. Figs. 7A and 7B, for example, show a keyboard 402 with a first portion 404 and a second portion 406 that provides haptic feedback during use. Fig. 7A shows a top view of the keyboard 402. The first portion 404 includes a first controller 408, a first battery 410, and a first transceiver 412. Piezoelectric stacks 414 on the first portion 404 are disposed under each character or key on the first portion 404. The second portion 406 includes a second controller 416, a second battery 418, and a second transceiver 420. Piezoelectric stacks 422 on the second portion 406 are disposed under each character or key of the second portion 406. Each piezoelectric stack 414, 422 includes or is adjacent to a vibrator 424.

In some embodiments the keyboard is made of glass or a transparent plastic. The transparent material improves an operator's view of the blood pumps.

Fig. 7B shows a side view of the keyboard 402. In this side view, it is easy to see the piezoelectric stacks 414, 422 that sense pressure and the vibrators 424. The piezoelectric stacks 414 and vibrators 424 on the first portion 404 are in electronic communication with the controller 408. The piezoelectric stacks 422 and vibrators 424 on the second portion 406 are in electronic communication with the second controller 416.

As explained above, each character on the keyboard 402 has a piezoelectric stack 414, 422 that determines if the operator has applied pressure to character. When the user presses a character on the first portion 404, the stack 414 sends a signal to the controller 408. The controller 408 then sends the character signal to the machine 100 using the transceiver 412 on the first portion 404 and sends a vibration signal to the vibrator 424 so that the pressed character vibrates. When the user presses a character on the second portion 406, the piezoelectric stack 422 sends a signal to the controller 416. The controller 416 then sends a character signal to the machine 100 using the transceiver 420 and sends a vibration signal to the vibrator 424 so that the pressed character vibrates.

In this embodiment, the first portion 404 and the second portion 406 operate independently. In this arrangement, if the first battery 410 is dead, the second portion 406 may operate as normal. In other embodiments, the transceiver 420 of the second portion 406 may only be in wireless communication with the transceiver 412 of the first portion 404. In such a case, all character signals are sent to the transceiver on the machine 100 from the transceiver 412 on the first portion 404. In this embodiment, the first portion 404 may operate independent from the second portion 406, but the second portion 406 may not operate independently from the first portion 404. Independently operating first and second positions may also be used in any other embodiment discussed previously or hereafter.

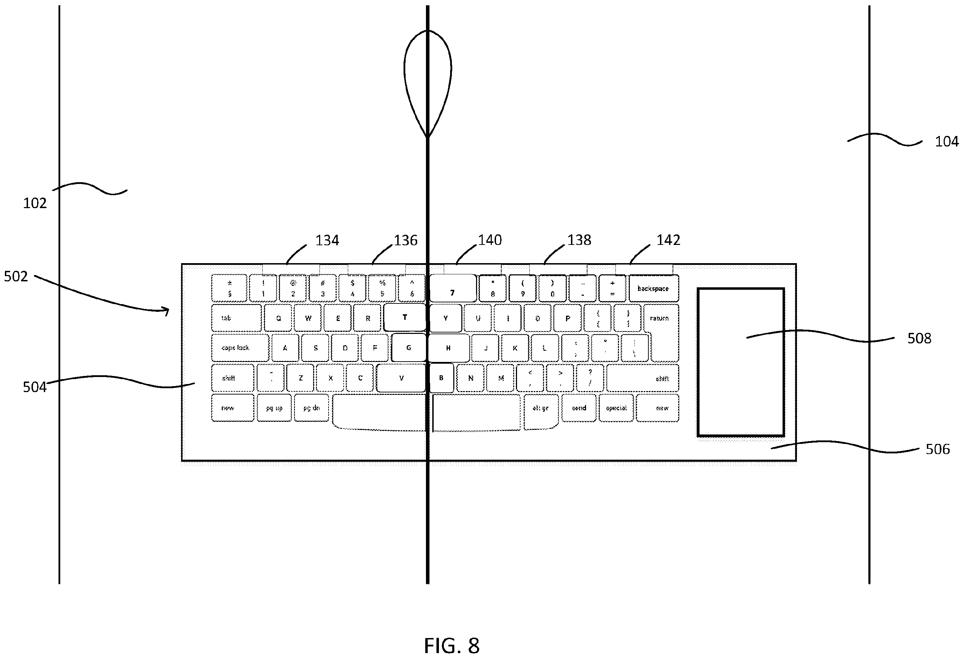

Fig. 8 shows an alternate embodiment of a keyboard 502 mounted on the first door 102 and the second door 104 of the machine 100. The keyboard 502 has a first portion 504 and a second portion 506. The second portion 506 includes a trackpad 508 that can be used as a mouse for selecting items on the user interface 114. In Fig. 8, the trackpad 508 shares the electronics of the second portion 506 of the keyboard 502. Both the first portion 504 and the second portion 506 house the electronics described with regard to Fig. 4. Similarly, the inputs of the trackpad 508, (e.g, moving the mouse, left click, right click, tapping, etc.) are sent from the transceiver 140 of the second portion 506 to the transceiver 116 of the machine.

While the keyboard 502 has been described as including a trackpad 508 to control movement of a cursor or other object on the user interface 114, other methods of moving such a cursor or other object may alternatively or additionally be included in the keyboard 502. For example, the keyboard 502 could use a joystick, roller ball, computer mouse, touch gesturing or trackpoint.

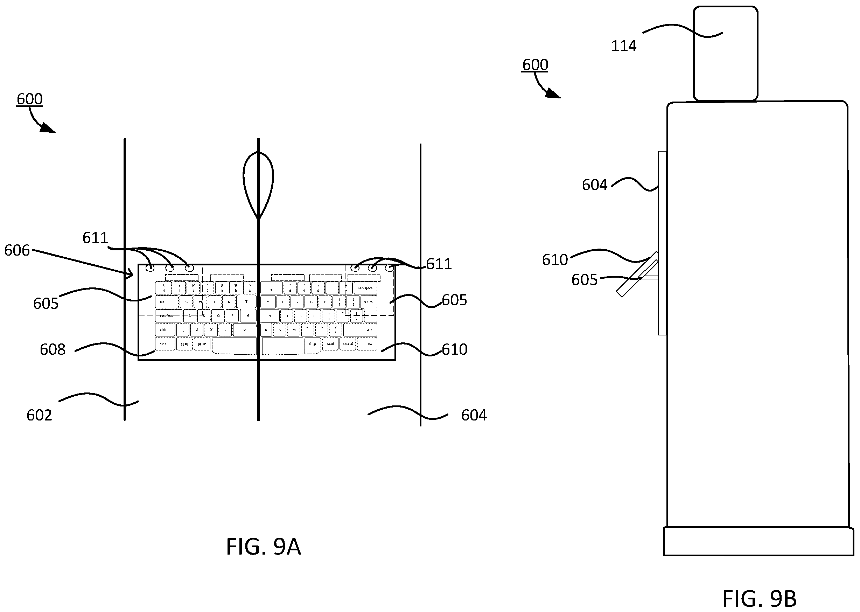

Figs. 9A and 9B show a blood treatment system 600, including the machine 100 with an alternate embodiment of a first door 602 and a second door 604. Doors 602, 604 are flat and do not contain a recess, as shown in the doors 102, 104 illustrated in Figs. 1-4. Rather, at least two brackets 605 are attached to the system 600, one bracket 605 on the first door 602 and one bracket 605 on the second door 604. In Fig. 9A, a keyboard 606 has a first portion 608 and a second portion 610. The first portion 608 is mounted on the bracket 605 attached to the first door 602. The second portion 610 is mounted on the bracket 605 attached to the second door 604. The brackets 605 are attached to the doors 602, 604 using bolts 611. In other embodiments, the brackets 605 are attached to the doors 602, 604 using any other fastener, such as screws, adhesive, or any other mating connector.

Fig. 9B shows a side view of the doors 602, 602 in the closed position. In this configuration the first portion 608 and the second portion 610 extend from the first door 602 and second door 604 at an angle. The keyboard 606 is shown at a 45° relative to the vertical doors 102, 104, however the keyboard may be positioned at any angle between 15° and 60°

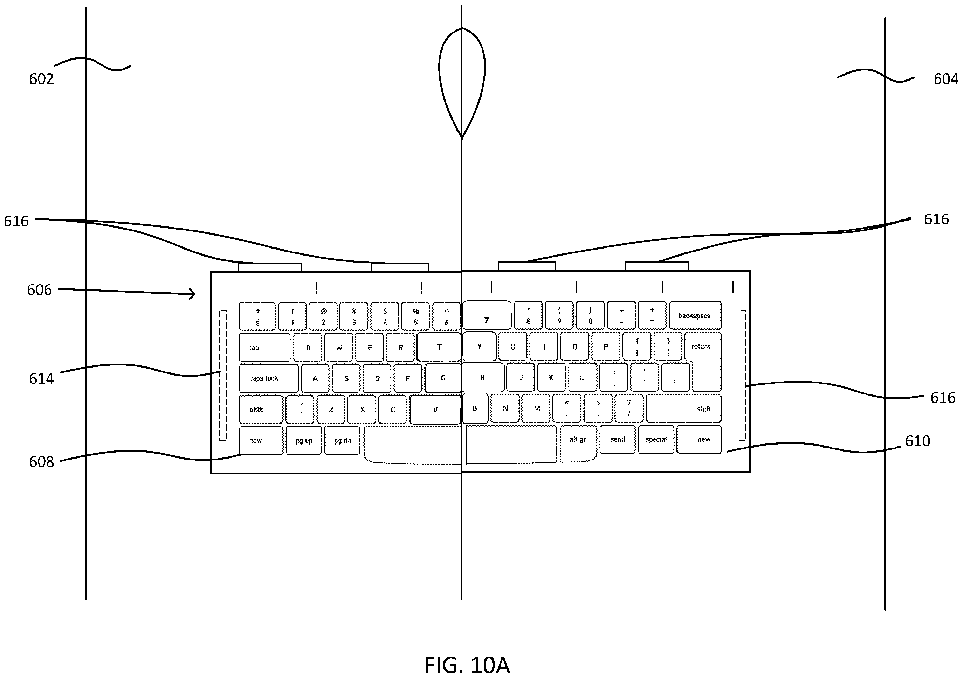

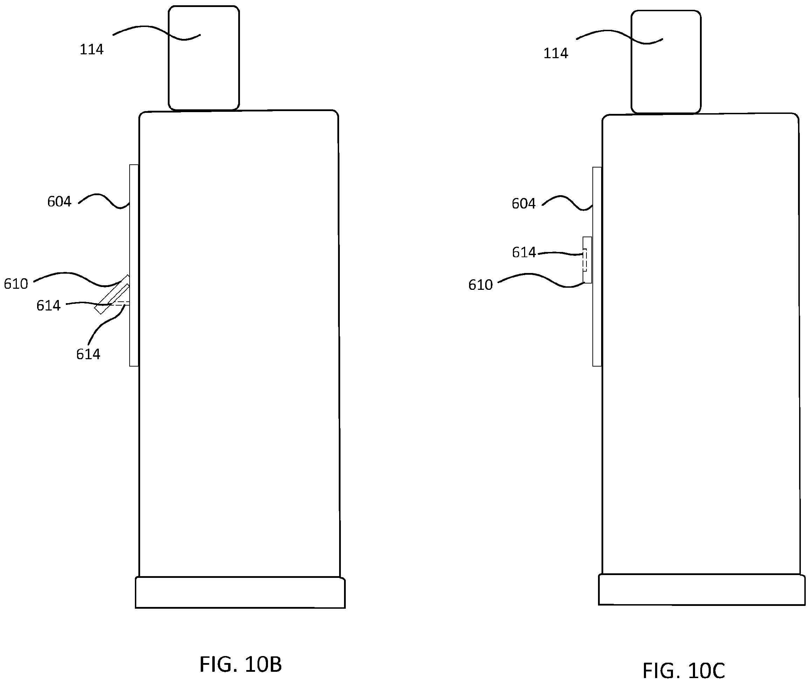

Figs. 10A-C shows the machine 100 with the keyboard 606 hingedly mounted on the doors 602, 604 of the machine 100. Figs. 10A and 10B show the keyboard 606 is in an expanded position, while Fig. 10C shows the keyboard 606 in a folded position. The first portion of the keyboard 606 is connected to the first door 602 by two hinges 612. The second portion 610 is mounted on the second door 604 by two hinges 612. In other embodiments the keyboard may be connected using one hinge or more than two hinges. The hinges 612 are linearly aligned to define an axis. The first portion 608 and the second portion rotate up about the axis to fold onto the first and second doors 602, 604 respectively. In the folded position, the first portion 608 and the second portion 610 are generally parallel with the doors 602, 604. A first support bar 614 and a second support bar 616 may be folded down from behind the keyboard 606 to add structural support to the keyboard 502 when an operator types.

Fig. 10B illustrates a side view the hinged split keyboard 606 in an expanded position. In the expanded position, the keyboard 606 can be used by an operator to input prescription data, patient data, or treatment notes. The support bars 614, 616 may be deployed to provide additional support when typing. The support bars 614, 616 extend from the back of the keyboard 606, and abut the doors 602, 604 of the machine. The hinges 612 may rotate up to 135° relative to the vertical doors 602, 604, so that the keyboard 606 is held at an angle when in the expanded position. In some embodiments, the support bars are of adjustable length so that the user can customize the tilt of the keyboard.

Fig. 10C illustrates a side view the hinged split keyboard 606 in a folded position. When the operator is finished typing, the operator may fold the keyboard up about the hinges 612 until the keyboard 606 is substantially parallel to the face of the doors 602, 604, or the keyboard 606 abuts the doors 602, 604.

In some embodiments, the hinges may allow for 180° rotation so that the keyboard 606 may fold up as described above, or may fold down so that the back of the keyboard is flat against the doors. To do this, a user retracts the support bars so that the keyboard is no longer supported by the support bars. In some embodiments the doors have cavities that are sized to receive the keyboard 606 in the folded position.

In alternate embodiments, the hinges may be mounted on outer edges of the keyboard 606. In such an embodiment the first portion rotates about a first vertical axis defined by the hinges that connect the first portion to the first door. The second portion rotates about a second vertical axis defined by the hinges that connect the second portion to the second door. In these embodiments, an operator can move the first portion and second portion of the keyboard about the vertical axis to better view the blood pumps. The axis defined by the hinges do not need to be vertical, but could also be set at an angle.

In another alternate embodiment, the first portion is connected to a movable arm that is mounted on the machine 100. The second portion is connected to a second arm that is mounted on the machine. The first and second arms may be mounted on the same section of the machine or may be mounted on different sections of the machine. For example, the first arm may be mounted on the left side of the machine and the second arm may be mounted on the right side of the machine. In other embodiments, a whole keyboard may be connected to an arm that is mounted on the machine. In either embodiment in which the keyboard or portions of the keyboard is are mounted on arms, the arms may include locks to prevent excess movement when typing.

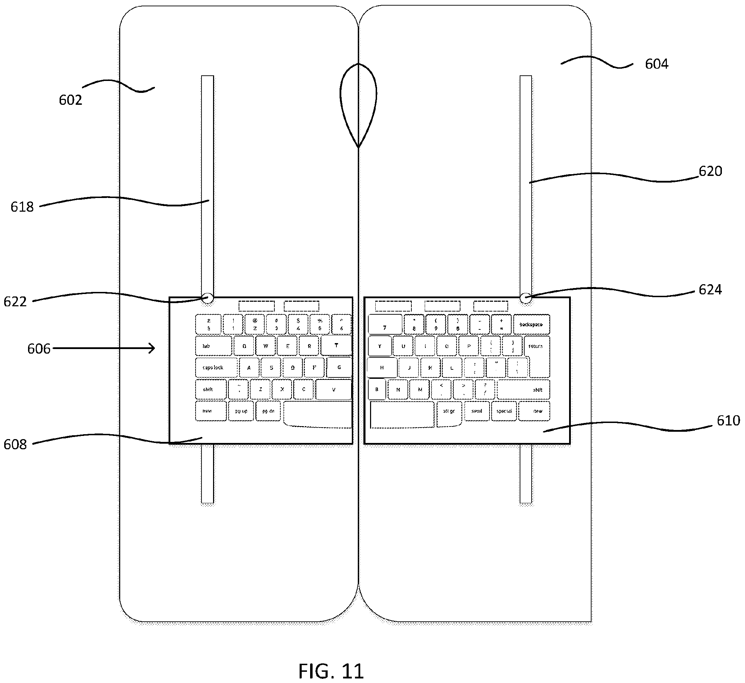

Fig. 11 shows the machine 100 with a first track 618 mounted on the first door 602 and a second track 620 mounted on the second door 604. The first portion 608 of the keyboard 606 is slidably connected to the first track 618 and the second portion 610 of the keyboard is slidably connected to the second track 620. Fig. 11 also shows a first lock 622 that locks the first portion 608 relative to the first track 618. A second lock 624 locks the second portion 610 relative to the second track 620. An operator manually unlocks and locks the first and second locks 622, 624 to adjust the height of the first and second portions 608, 610. The height of the keyboard 606 is measured from the top edge keyboard 606. For example, the height of the keyboard 606 in Figure 11 is measured from the edge of the keyboard 606, near the first lock 622 for the first portion, and near the second lock 624 for the second portion. The maximum height of the keyboard 606 can be between 122 cm (48 inches) and 107 cm (42 inches). The minimum height of the keyboard 606 can be between 91 cm (36 inches) and 5.1 cm (32 inches). These maximum and minimum heights are suitable for operators of heights between 193 cm (six feet and four inches) and 135 cm (4 feet and five inches). The adjustable keyboard 606 can be moved to accommodate users of different heights, increasing user comfort.

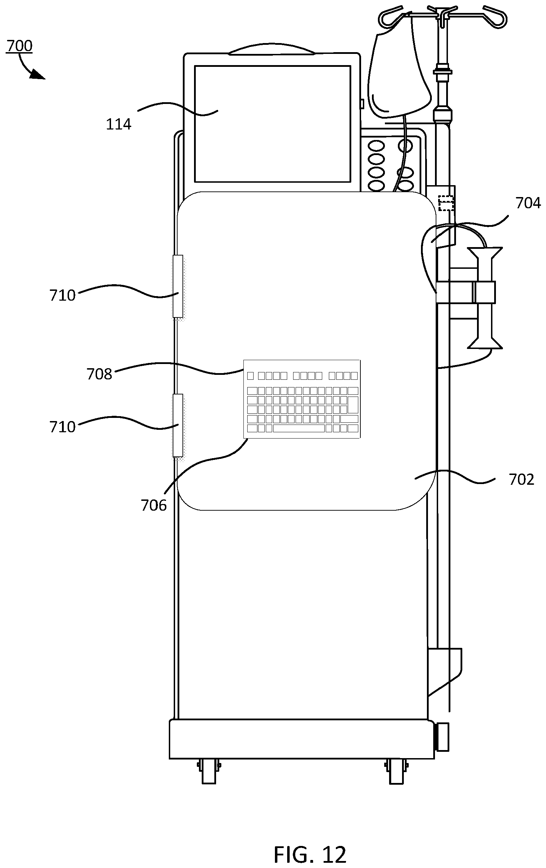

Fig. 12 shows a blood treatment system 700 including the machine 100 with a single door 702. The blood treatment machine 100 is the same machine as shown in Fig. 1. However, the system in Fig. 12 has a single door 702 with a handle 704. The door 702 has a ledge 706. A full keyboard 708 is mounted on the ledge 706. The door 702 is in the closed position. In this position blood treatment may commence. The door 702 can be opened using the handle 704. The door 702 is attached to the machine 100 by hinges 710 that define an axis. When moving from the closed position to an open position, the door 702 rotates about the axis.

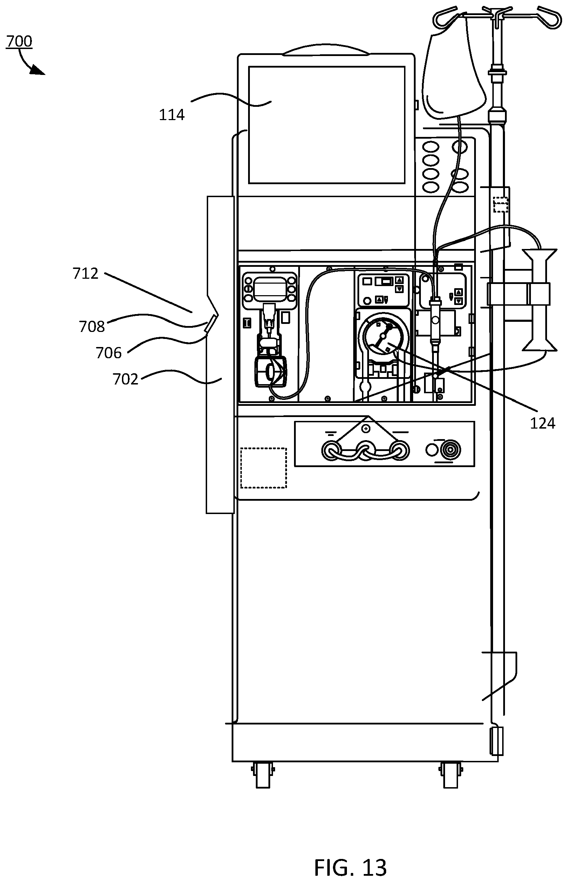

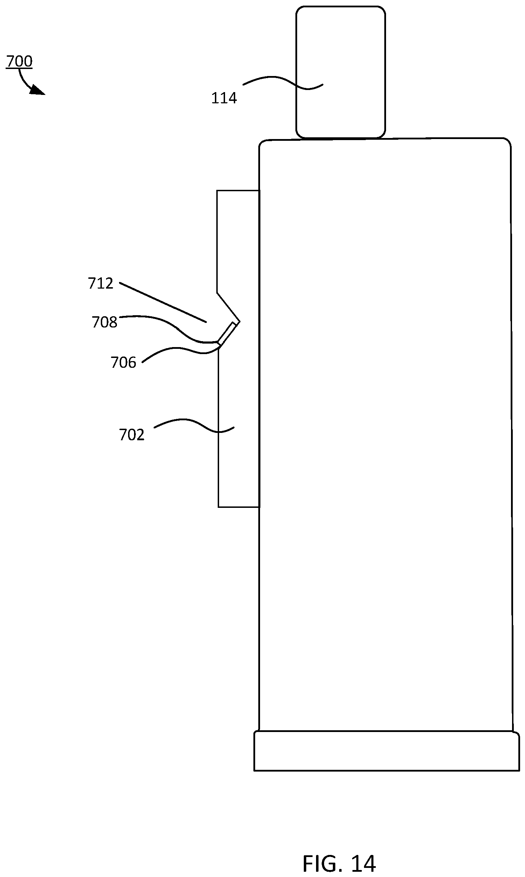

Fig. 13 shows the door 702 of the system 700 in the open position. The keyboard 708 moves with the door 702 so that the keyboard 708 is out of the way and the operator is able to access the pumps and bloodlines. In the open position, the machine 100 is prevented from providing blood treatment to the patient. In some embodiments, the open and closed positions are detected by a latch sensor. Treatment can be prevented when the latch sensor detects open door(s). Fig. 14 shows a side view of the system 700 with the door 702 in the closed position. The ledge 706 extends into the door 702 towards the machine 100 at an angle. The angle may be between 15° and 60°. The ledge 706 partially defines a recess 712 in the door 702. The recess 712 is sized to receive hands of the operator for typing. Typing applies repeated forces onto a keyboard, in addition to the weight of the keyboard and the weight of the hands of the operator. The keyboard 708 is supported by mounting the keyboard in the recess 712 of the door 702. In such a configuration, the keyboard 708 does not deform over time due to the repeatedly applied forces and does not significantly bounce or shift during typing.

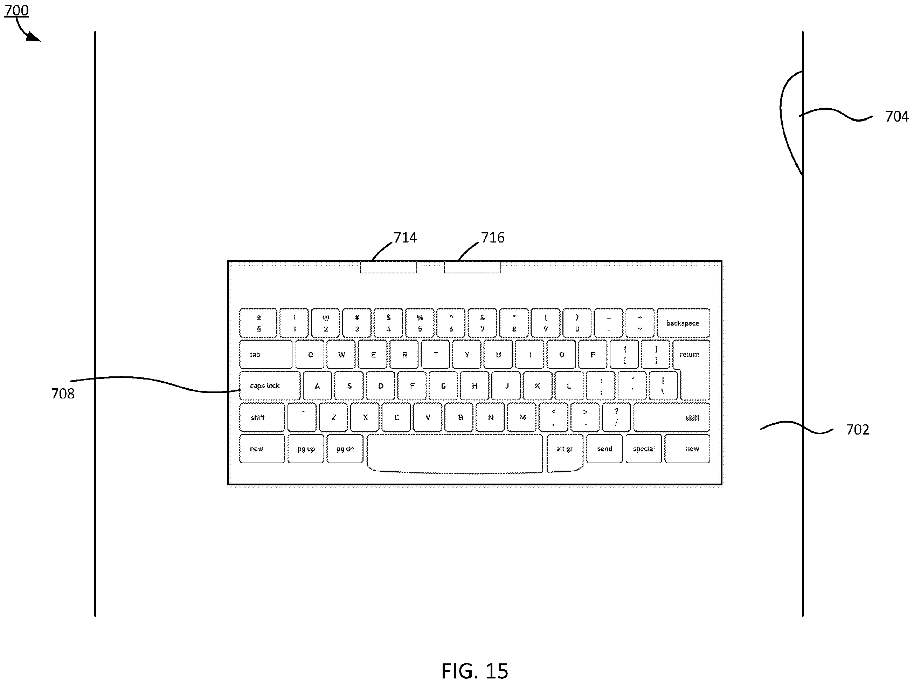

Fig. 15 shows a close up of the keyboard 708. The keyboard 708 includes a battery 714 and a transceiver 716. The transceiver 716 is in wireless communication with the transceiver 136 of the machine 100. The transceiver 716 of the keyboard 708 is configured to send keystroke signals to the transceiver 116 of the machine 100. The controller 115 of the machine 100 processes the keystroke signal and displays a character assigned to the signal on the user interface 114. In other alternative embodiments an electronic connecter may be attached to the first portion of the keyboard. The second portion of the keyboard has a receiving cavity for the connector, so that, when the doors are in the closed position, the first and second portion may be latched together using the electronic connector. The electronic connecter is configured to electronically connect the first and second portion together so that they share the same transceiver, battery, and processor. The electronic connector could also be attached to the second portion. In such a case, the first portion has a cavity to receive the electronic connector.

While transceivers have been described as being integrated into the machine, in some embodiments the signal transceiver on the machine is a detachable transceiver such as a USB insert.

While the second portion of the keyboard has been described having a second transceiver in wireless communication with the transceiver of the machine, other embodiments, have both the first transceiver and the second transceiver in wireless communication to the transceiver of the machine. In such cases, the first portion and second portion may operate independently, each portion sending a keystroke signal to the transceiver of the machine. The processor of the machine then processes the keystroke signal and orders multiple keystroke signals based on a timestamp applied to the keystroke signal. This timestamp may be applied to the keystroke signal by the transceiver of the portion of the keyboard when the signal is transmitted or may be applied by the transceiver of the machine when the keystroke signal is received by the machine. The controller then displayed the character associated with the keystroke signal on the user interface.

While keyboards having letter keys and touchpads have been discussed, the keyboard can alternatively or additionally include other features in some embodiments, for example, the layout of the keyboard may include a number pad. The keyboards described above are on hemodialysis machines, but other blood treatment machines, including peritoneal dialysis machines can include similar keyboards. Similar keyboards may also be included in other types of medical devices.

While the above keyboards have been described as being wirelessly connected to the machine, in some embodiments, the keyboard may be hardwired into the machine 100, for example, the keyboard, can be connected to the machine via wires that travel through the hinges of the doors.

Accordingly, other embodiments are within the scope of the following claims.

## Claims

1. A blood treatment machine comprising:
a user interface (114) configured to display information;
a transceiver (116) configured to send and receive signals,
a controller (115) configured to process input signals received by the transceiver and display information on the user interface,
one or more pumps (124),
a first door (102, 104, 602, 604, 702) having a closed configuration in which the door covers the one or more pumps and an open configuration in which the one or more pumps are accessible by a user, and
at least a portion of a keyboard (106, 302, 402, 502, 606, 708) mounted on the door, the keyboard being configured to transmit signals to the transceiver of the machine.

2. The blood treatment machine according to claim 1, wherein the blood treatment machine comprises a surface on which the one or more pumps are disposed-, and optionally wherein the door covers the surface.

3. The blood treatment machine according to any of the preceding claims, wherein the door is a first door (102, 104, 602, 604) and the blood treatment machine further comprises a second door (102, 104, 602, 604).

4. The blood treatment machine according to claim 3, wherein the portion of the keyboard mounted on the door is a first portion (108, 110, 304, 308, 404, 406, 504, 506, 608, 610) mounted on the first door and the keyboard has a second portion (108, 110, 304, 308, 404, 406, 504, 506, 608, 610) mounted on the second door.

5. The blood treatment machine according to claim 4, wherein the first portion of the keyboard and the second portion of the keyboard are wirelessly connected.

6. The blood treatment machine according to claim 4 or 5, wherein the first portion of the keyboard comprises a signal transceiver (140), a battery (138), and a controller (142) and the second portion of the keyboard comprises a signal transceiver (134) and a battery (136).

7. The blood treatment machine according to claim 6, wherein the transceiver of the first portion of the keyboard is configured to send signals to the transceiver of the blood treatment machine.

8. The blood treatment machine according to any of the preceding claims, wherein either the keyboard is bolted onto the door or the keyboard is mounted on a bracket.

9. The blood treatment machine according to any of the preceding claims, wherein the door comprises a recess (120, 122, 712) and a ledge (112, 113, 706) that defines a portion of the recess, wherein the keyboard is mounted on the ledge.

10. The blood treatment machine according to any of the preceding claims,
wherein either:
the keyboard is wirelessly connected to the machine, or
the keyboard is electronically connected to the machine using wired connections.

11. The blood treatment machine according to any of the preceding claims,
wherein the transceiver is releasably connected to the machine, and
optionally wherein the transceiver is connected to the machine by a USB port.

12. The blood treatment machine according to any of the preceding claims,
wherein the keyboard is configured to transmit signals to the transceiver of the machine using bluetooth.

13. The blood treatment machine according to any of the preceding claims,
wherein the keyboard is on a front face of the door, accessible to a user in the closed configuration.

14. A system in which a blood treatment machine is configured to communicate with a remote server (200), the system comprising:
the blood treatment machine according to any preceding claim, wherein:
the controller (115) is also configured to control the blood treatment machine,
the keyboard is electronically connected to the blood treatment machine,; and
the remote server is in connection with the blood treatment machine,
wherein the remote server contains patient information and the blood treatment machine is configured to retrieve patient information from the remote server using inputs received from the keyboard.

15. The system according to claim 14, wherein the remote server and machine are in wireless connection and the keyboard and the machine are in wireless connection.

## Patentansprüche

1. Blutbehandlungsmaschine, umfassend:
eine Benutzerschnittstelle (114), die ausgelegt ist, Informationen anzuzeigen;
einen Sendeempfänger (116), der ausgelegt ist, Signale zu senden und zu empfangen,
eine Steuereinheit (115), die ausgelegt ist, Eingangssignale, die durch den Sendeempfänger erhalten werden, zu verarbeiten, und Informationen an der Benutzerschnittstelle anzuzeigen,
eine oder mehrere Pumpen (124),
eine erste Tür (102, 104, 602, 604, 702), die eine geschlossene Konfiguration, in der die Tür die eine oder die mehreren Pumpen bedeckt, und eine offene Konfiguration, in der die eine oder die mehreren Pumpen für einen Benutzer zugänglich sind, hat, und
wenigstens einen Abschnitt einer Tastatur (106, 302, 402, 502, 606, 708), der an der Tür montiert ist, wobei die Tastatur ausgelegt ist, Signale an den Sendeempfänger der Maschine zu senden.

2. Blutbehandlungsmaschine nach Anspruch 1, wobei die Blutbehandlungsmaschine eine Oberfläche besitzt, auf der die eine oder die mehreren Pumpen angeordnet sind, und wobei die Tür optional die Oberfläche bedeckt.

3. Blutbehandlungsmaschine nach einem der vorhergehenden Ansprüche, wobei die Tür eine erste Tür (102, 104, 602, 604) ist und die Blutbehandlungsmaschine ferner eine zweite Tür (102, 104, 602, 604) umfasst.

4. Blutbehandlungsmaschine nach Anspruch 3, wobei der Abschnitt der Tastatur, der an der Tür montiert ist, ein erster Abschnitt (108, 110, 304, 308, 404, 406, 504, 506, 608, 610) ist, der an der ersten Tür montiert ist, und die Tastatur einen zweiten Abschnitt (108, 110, 304, 308, 404, 406, 504, 506, 608, 610) besitzt, der an der zweiten Tür montiert ist.

5. Blutbehandlungsmaschine nach Anspruch 4, wobei der erste Abschnitt der Tastatur und der zweite Abschnitt der Tastatur drahtlos verbunden sind.

6. Blutbehandlungsmaschine nach Anspruch 4 oder 5, wobei der erste Abschnitt der Tastatur einen Signal-Sendeempfänger (140), eine Batterie (138) und eine Steuereinheit (142) umfasst und der zweite Abschnitt der Tastatur einen Signal-Sendeempfänger (134) und eine Batterie (136) umfasst.

7. Blutbehandlungsmaschine nach Anspruch 6, wobei der Sendeempfänger des ersten Abschnitts der Tastatur ausgelegt ist, Signale an den Sendeempfänger der Blutbehandlungsmaschine zu senden.

8. Blutbehandlungsmaschine nach einem der vorhergehenden Ansprüche, wobei die Tastatur an der Tür verschraubt ist oder die Tastatur an einer Halterung montiert ist.

9. Blutbehandlungsmaschine nach einem der vorhergehenden Ansprüche, wobei die Tür eine Vertiefung (120, 122, 712) und einen Absatz (112, 113, 706), der einen Abschnitt der Vertiefung definiert, umfasst, wobei die Tastatur am Absatz montiert ist.

10. Blutbehandlungsmaschine nach einem der vorhergehenden Ansprüche, wobei:
die Tastatur mit der Maschine drahtlos verbunden ist, oder
die Tastatur mit der Maschine unter Verwendung von drahtgebundenen Verbindungen elektronisch verbunden ist.

11. Blutbehandlungsmaschine nach einem der vorhergehenden Ansprüche, wobei der Sendeempfänger mit der Maschine lösbar verbunden ist, und
wobei der Sendeempfänger optional mittels eines USB-Anschlusses mit der Maschine verbunden ist.

12. Blutbehandlungsmaschine nach einem der vorhergehenden Ansprüche, wobei die Tastatur ausgelegt ist, Signale an den Sendeempfänger der Maschine unter Verwendung von Bluetooth zu senden.

13. Blutbehandlungsmaschine nach einem der vorhergehenden Ansprüche, wobei die Tastatur auf einer Vorderseite der Tür angeordnet ist, so dass sie in der geschlossenen Konfiguration für einen Benutzer zugänglich ist.

14. System, in dem eine Blutbehandlungsmaschine ausgelegt ist, mit einem entfernten Server (200) zu kommunizieren, wobei das System Folgendes umfasst:
die Blutbehandlungsmaschine nach einem der vorhergehenden Ansprüche, wobei:
die Steuereinheit (115) auch ausgelegt ist, die Blutbehandlungsmaschine zu steuern,
die Tastatur mit der Blutbehandlungsmaschine elektronisch verbunden ist; und
der entfernte Server mit der Blutbehandlungsmaschine in Verbindung ist,
wobei der entfernte Server Patienteninformationen enthält und die Blutbehandlungsmaschine ausgelegt ist, Patienteninformationen vom entfernten Server unter Verwendung von Eingangssignalen, die von der Tastatur empfangen werden, abzurufen.

15. System nach Anspruch 14, wobei der entfernte Server und die Maschine drahtlos verbunden sind und die Tastatur und die Maschine drahtlos verbunden sind.

## Revendications

1. Machine de traitement du sang comportant :
une interface (114) d'utilisateur configurée pour afficher des informations ;
un émetteur-récepteur (116) configuré pour émettre et recevoir des signaux,
un dispositif (115) de commande configuré pour traiter des signaux d'entrée reçus par l'émetteur-récepteur et afficher des informations sur l'interface d'utilisateur, une ou plusieurs pompes (124),
une première porte (102, 104, 602, 604, 702) possédant une configuration fermée dans laquelle la porte recouvre la ou les pompes et une configuration ouverte dans laquelle la ou les pompes sont accessibles par un utilisateur, et
au moins une partie d'un clavier (106, 302, 402, 502, 606, 708) montée sur la porte, le clavier étant configuré pour transmettre des signaux à l'émetteur-récepteur de la machine.

2. Machine de traitement du sang selon la revendication 1, la machine de traitement du sang comportant une surface sur laquelle la ou les pompes sont disposées, et la porte recouvrant optionnellement la surface.

3. Machine de traitement du sang selon l'une quelconque des revendications précédentes, la porte étant une première porte (102, 104, 602, 604) et la machine de traitement du sang comportant en outre une seconde porte (102, 104, 602, 604).

4. Machine de traitement du sang selon la revendication 3, la partie du clavier montée sur la porte étant une première partie (108, 110, 304, 308, 404, 406, 504, 506, 608, 610) montée sur la première porte et le clavier comprenant une seconde partie (108, 110, 304, 308, 404, 406, 504, 506, 608, 610) montée sur la seconde porte.

5. Machine de traitement du sang selon la revendication 4, la première partie du clavier et la seconde partie du clavier étant reliées sans fil.

6. Machine de traitement du sang selon la revendication 4 ou 5, la première partie du clavier comportant un émetteur-récepteur (140) de signaux, une batterie (138) et un dispositif de commande (142), et la seconde partie du clavier comportant un émetteur-récepteur (134) de signaux et une batterie (136).

7. Machine de traitement du sang selon la revendication 6, l'émetteur-récepteur de la première partie du clavier étant configuré pour envoyer des signaux à l'émetteur-récepteur de la machine de traitement du sang.

8. Machine de traitement du sang selon l'une quelconque des revendications précédentes, soit le clavier étant boulonné sur la porte, soit le clavier étant monté sur un support.

9. Machine de traitement du sang selon l'une quelconque des revendications précédentes, la porte comportant un évidement (120, 122, 712) et un rebord (112, 113, 706) qui définit une partie de l'évidement, le clavier étant monté sur le rebord.

10. Machine de traitement du sang selon l'une quelconque des revendications précédentes :
soit le clavier étant relié sans fil à la machine,
soit le clavier étant relié électroniquement à la machine à l'aide de liaisons filaires.

11. Machine de traitement du sang selon l'une quelconque des revendications précédentes, l'émetteur-récepteur étant lié à la machine de manière détachable, et l'émetteur-récepteur étant optionnellement relié à la machine par un port USB.

12. Machine de traitement du sang selon l'une quelconque des revendications précédentes, le clavier étant configuré pour transmettre des signaux à l'émetteur-récepteur de la machine par Bluetooth.

13. Machine de traitement du sang selon l'une quelconque des revendications précédentes, le clavier se trouvant sur une face avant de la porte, accessible à un utilisateur dans la configuration fermée.

14. Système dans lequel une machine de traitement du sang est configurée pour communiquer avec un serveur distant (200), le système comportant :
la machine de traitement du sang selon l'une quelconque des revendications précédentes :
le dispositif (115) de commande étant également configuré pour commander la machine de traitement du sang,
le clavier étant relié électroniquement à la machine de traitement du sang, et
le serveur distant étant en liaison avec la machine de traitement du sang,
le serveur distant contenant des informations de patient et la machine de traitement du sang étant configurée pour récupérer des informations de patient à partir du serveur distant en utilisant des entrées reçues en provenance du clavier.

15. Système selon la revendication 14, le serveur distant et la machine étant en liaison sans fil et le clavier et la machine étant en liaison sans fil.
